# EUROPEAN PATENT APPLICATION

(11) **EP 4 755 310 A1**
(43) Date of publication of application: **10.06.2026**
(21) Application number: 25218789.3
(22) Date of filing: 26.11.2025
(51) Int. Cl.: A61B 8/00

(54) **ULTRASOUND DIAGNOSTIC APPARATUS**

(30) Priority: 06.12.2024 JP 2024213195
(71) Applicant: FUJIFILM Corporation, Tokyo 106-8620 (JP)
(72) Inventor: KUNIEDA, Shuji, Tokyo, 106-8620 (JP); KASAHARA, Eiji, Tokyo, 106-8620 (JP)
(74) Representative: Meissner Bolte Partnerschaft mbB

(57) **Abstract**

An object is to receive designation of a two-dimensional position on an ultrasound image and enable control of an ultrasound diagnostic apparatus in accordance with the designated position.

A display unit receives designation of a point on a displayed ultrasound image by a touch operation or the like. A positional information conversion unit converts coordinates of the point expressed in a coordinate system of the ultrasound image into coordinates in a coordinate system used by an ultrasound diagnostic apparatus for ultrasound beam scanning. A central controller and a control position setting unit control the ultrasound diagnostic apparatus (for example, setting an ROI) using the coordinates obtained by the coordinate conversion.

## Description

### BACKGROUND OF THE INVENTION

### 1. Field of the Invention

The present invention relates to an ultrasound diagnostic apparatus.

### 2. Description of the Related Art

In an ultrasound diagnostic apparatus in the related art, a position of an object such as a sample volume for pulsed Doppler display or a region of interest (ROI) for color Doppler display has been changed by an operation of a trackball or arrow keys. This position change has been executed by a method for moving an operation point by an amount corresponding to an operation amount of the trackball or the like from a current position in a direction indicated by the trackball or the like in a scanning coordinate system of an ultrasound beam.

The scanning coordinate system is defined by a depth direction, which is a direction in which the ultrasound beam travels, and a scanning direction in which scanning with the ultrasound beam is performed. For example, in a case in which a user pushes a button for giving an instruction to change the position of the sample volume on an operation panel and presses a right (or left) arrow key once, the position of the sample volume on the screen is moved from the current position by one step to the right (or left) along the scanning direction. A movement amount of the sample volume is an amount corresponding to the number of times the right arrow key is pressed. The up and down arrow keys are used to give an instruction of the movement in the depth direction. The same applies to the operation using the trackball. In a case in which the trackball is rotated to the right, the sample volume is moved from the current position by an amount corresponding to the rotation amount to the right along the scanning direction.

In addition, in recent years, apparatuses that use a touch panel as a screen for displaying an ultrasound image have become widespread (for example, JP2006-026256A). By using the touch panel, the position on the ultrasound image can be intuitively and easily designated as compared to a mouse or a trackball.

An ultrasound diagnostic apparatus disclosed in JP2009-207589A is designed to facilitate linear input in a vertical or horizontal direction along sides by providing touch panel regions along four sides of an outer frame of a monitor.

An ultrasound diagnostic apparatus disclosed in JP2016-214650A has swipe regions on four sides of an outer periphery of a display region of an ultrasound image, and changes a set value of a function assigned to the swipe region in response to a touch operation of a user on the swipe region.

An ultrasound diagnostic apparatus disclosed in JP2012-019824A displays a touch button near a marker indicating an operation target point in an ultrasound image, and moves, in a case in which a user moves the touch button by a touch operation, the marker in response to the touch operation.

An ultrasound image diagnostic apparatus disclosed in JP2015-198810A displays, in a case in which a measurement cursor on an ultrasound image displayed on a touch panel screen is moved by a touch operation, a button for giving an instruction to confirm a position of the cursor at an end point of the movement, near the cursor at the end point.

An ultrasound image diagnostic apparatus disclosed in JP2006-296978A aligns a vertical coordinate of a touch panel monitor that displays an ultrasound image with a depth direction coordinate of the ultrasound image, and associates a horizontal coordinate with an STC gain for adjusting the ultrasound image. In a case in which a user touches a desired position on the touch panel with a finger and drags the finger horizontally or diagonally, the apparatus adjusts the STC gain at the position touched by the finger in real time.

JP2009-056202A discloses an ultrasound diagnostic apparatus that changes a focus depth, a position, or a size of an ROI in response to a touch operation on a touch panel.

The method for moving the object by the operation amount in a direction indicated by the operation of the trackball or the like from the current position is inferior in operability. For example, a case will be considered in which the user finds a blood vessel on the ultrasound image on the screen and wants to set the sample volume to the blood vessel. In this case, gradually moving the sample volume from the current position to the position of the blood vessel by rotating the trackball in the vertical or horizontal direction is a time-consuming operation.

There are other cases in which a position or the like of a point or a range (for example, the sample volume or the ROI) used for controlling the ultrasound diagnostic apparatus is designated by the operation of the trackball or the like, and in these other cases, similar problems can occur.

Among the related-art apparatuses that display the ultrasound image on the touch panel, there is an apparatus that receives the designation of the position in the ultrasound image by the touch operation. However, in most cases, the position designation by the touch operation is used for measurement (for example, measurement of a distance between two points) on the ultrasound image using the designated position.

In addition, the related-art apparatus that associates the position in the vertical direction of the touch panel with the depth direction position of the ultrasound image and associates the position in the horizontal direction with the value of the signal gain receives the designation of the position in the depth direction, but does not receive the designation of the two-dimensional position.

### SUMMARY OF THE INVENTION

The present specification discloses an ultrasound diagnostic apparatus comprising: a processor configured to: receive information on a point designated on an ultrasound image displayed on a screen; perform coordinate conversion of coordinates of the designated point in a display coordinate system of the ultrasound image that are indicated by the information into coordinates in a scanning coordinate system of an ultrasound beam; and execute control of the ultrasound diagnostic apparatus using the coordinates of the designated point that have undergone the coordinate conversion.

In one aspect, the processor may be configured to: in the control, set a range or a position that is used to generate the ultrasound image based on the coordinates of the designated point that have undergone the coordinate conversion.

In particular, the range or the position that is used to generate the ultrasound image and that is a target of the setting may define a transmission range or a transmission position of the ultrasound beam for a specific display mode of the ultrasound diagnostic apparatus.

The range that is used to generate the ultrasound image and that is a target of the setting may define a range of reception signals subjected to calculation for generating an image of a specific display mode of the ultrasound diagnostic apparatus.

The range that is used to generate the ultrasound image and that is the target of the setting may be a sample volume for pulsed Doppler display.

The range that is used to generate the ultrasound image and that is the target of the setting may be an ROI that is a range for color Doppler display.

The range that is used to generate the ultrasound image and that is the target of the setting may be a scanning range with the ultrasound beam for B-mode tomographic image display.

The processor may be configured to: set the range that is used to generate the ultrasound image based on the designated point on the ultrasound image displayed on the screen.

In one aspect, the processor may be configured to: receive information on two points designated on the ultrasound image displayed on the screen; and enlarge or reduce the range in a case in which a distance between the two designated points is increased or decreased over time.

The present specification further discloses a program causing a computer to execute a process comprising: receiving information on a point designated on an ultrasound image displayed on a screen; performing coordinate conversion of coordinates of the designated point in a display coordinate system of the ultrasound image that are indicated by the information into coordinates in a scanning coordinate system of an ultrasound beam; and executing control of the ultrasound diagnostic apparatus using the coordinates of the designated point that have undergone the coordinate conversion.

With the above-described configuration, the two-dimensional position on the ultrasound image can be designated, and the ultrasound diagnostic apparatus can be controlled in accordance with the designated position.

The present specification further discloses a method for controlling an ultrasound diagnostic apparatus comprising: receiving information on a point designated on an ultrasound image displayed on a screen; performing coordinate conversion of coordinates of the designated point in a display coordinate system of the ultrasound image that are indicated by the information into coordinates in a scanning coordinate system of an ultrasound beam; and executing control of the ultrasound diagnostic apparatus using the coordinates of the designated point that have undergone the coordinate conversion.

### BRIEF DESCRIPTION OF THE DRAWINGS

FIG. 1 is a block diagram showing a configuration of an ultrasound diagnostic apparatus according to an embodiment.
FIG. 2 is a diagram showing an example of a processing procedure of the embodiment.
FIG. 3 is a diagram showing a detailed procedure of step S26 of the procedure shown in FIG. 2.
FIG. 4 is a diagram showing a part of an example of processing of specifying an object as an operation target in response to a touch operation.
FIG. 5 is a diagram showing a remaining part of the example of the processing of specifying the object as the operation target in response to the touch operation.
FIG. 6 is a diagram showing an operation of moving a cursor of a sample volume by the touch operation.
FIG. 7 is a diagram showing an operation of changing a width of an ROI by the touch operation.
FIG. 8 is a diagram showing a transmit beam focus in a depth direction and in a scanning direction.
FIG. 9 is a block diagram showing a configuration of a modification example in which an external device comprising a touch panel displays an ultrasound image.

### DESCRIPTION OF THE PREFERRED EMBODIMENTS

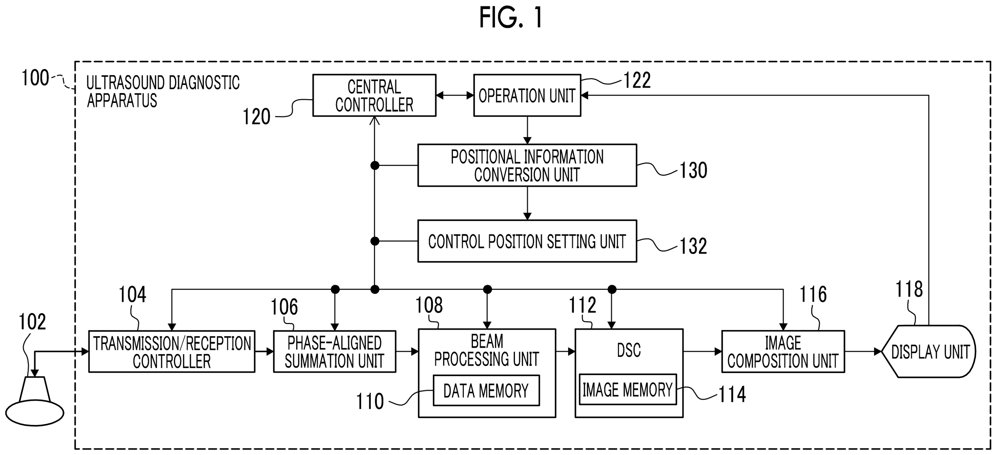

FIG. 1 shows an example of a configuration of an ultrasound diagnostic apparatus 100 according to an embodiment of the present disclosure.

A probe 102 is a device that transmits and receives ultrasound beams for ultrasound diagnosis. The probe 102 includes a transducer element array configured by arranging a plurality of transducer elements. Each transducer element converts between an electrical signal and an ultrasound signal by the piezoelectric effect. There are several types of probe 102, such as a linear type, a sector type, and a convex type.

A transmission/reception controller 104 controls the transmission and reception of the ultrasound by each transducer element in the probe 102. The control includes, for example, supply of an electrical transmission signal to each transducer element or amplification of an electrical reception signal from each transducer element. In the supply of the transmission signal, the transmission/reception controller 104 forms a transmit ultrasound beam by controlling a supply timing of the transmission signal to each transducer element.

A phase-aligned summation unit 106 executes phase-aligned summation processing on the reception signal from each transducer element in the probe 102. A reception beam is formed by the phase-aligned summation processing. The phase-aligned summation unit 106 outputs echo data obtained along the reception beam as a result of the phase-aligned summation processing.

A beam processing unit 108 executes various types of signal processing, such as gain correction processing, logarithmic amplification processing, envelope detection processing, and filter processing, on the echo data output by the phase-aligned summation unit 106. The beam processing unit 108 executes the signal processing using a data memory 110 as a working memory. As a result, beam data corresponding to each echo data is formed.

A digital scan converter (DSC) 112 has a coordinate conversion function and an interpolation function, and forms a display frame, that is, an ultrasound image based on a plurality of beam data output from the beam processing unit 108. The beam data from the beam processing unit 108 is data in a beam scanning coordinate system (hereinafter, referred to as a "scanning coordinate system"), and is composed of a plurality of data points along a direction of the beam corresponding to the beam data. The DSC 112, for example, plots a signal value of each data point of the beam data at a position of the data point in a display coordinate system, that is, a coordinate system of the ultrasound image (in general, a Cartesian coordinate system indicated by a set of an x coordinate and a y coordinate). The plotting is executed by writing data to the image memory 114 that holds the signal value of each pixel in accordance with the display coordinate system. The DSC 112 interpolates the values of pixels that do not yet have values in the image memory 114 after the beam data is written, from the values of the surrounding pixels. The ultrasound image such as a B-mode tomographic image is formed by the coordinate conversion and the interpolation.

The image composition unit 116 combines and displays an image or a character indicating various types of information with the ultrasound image formed by the DSC 112 to form display screen data. Examples of the information to be combined with the ultrasound image include an ROI representing a display range of various display modes such as a color Doppler mode, a sample volume of the pulsed Doppler mode, and a line indicating a beam on which the sample volume is located.

A display unit 118 is a device that displays an image, and is composed of, for example, a liquid-crystal panel or an organic EL panel. The display screen data formed by the image composition unit 116 is displayed on the display unit 118.

A central controller 120 controls the operation of each unit of the ultrasound diagnostic apparatus 100 to implement the function of the ultrasound diagnostic apparatus 100. The central controller 120 includes, as hardware, for example, a processor such as a CPU or a microcontroller, a memory that functions as a primary storage, and a large-capacity storage device that functions as a secondary storage. The central controller 120 controls the ultrasound diagnostic apparatus 100 by executing a program stored in the secondary storage.

An operation unit 122 is a device that receives an operation of the ultrasound diagnostic apparatus 100 from a user. The operation unit 122 may comprise mechanical input elements such as a keyboard, a button, a switch, and a knob. Further, the operation unit 122 may comprise a pointing device such as a trackball or a mouse.

Furthermore, the operation unit 122 may comprise a touch panel provided on a display screen of the display unit 118. The information on a touch position with a finger of the user detected by the touch panel or a change in the touch position is interpreted by the central controller 120 and is used in the ultrasound diagnostic apparatus 100.

In the control or the processing executed by the central controller 120, there is processing that is executed in the scanning coordinate system. A representative example of the control executed in the scanning coordinate system is control related to the transmission and reception of the ultrasound.

Examples of the control related to the ultrasound transmission and reception include setting of the sample volume in the pulsed Doppler mode. The pulsed Doppler mode is also called a pulse wave (PW) mode.

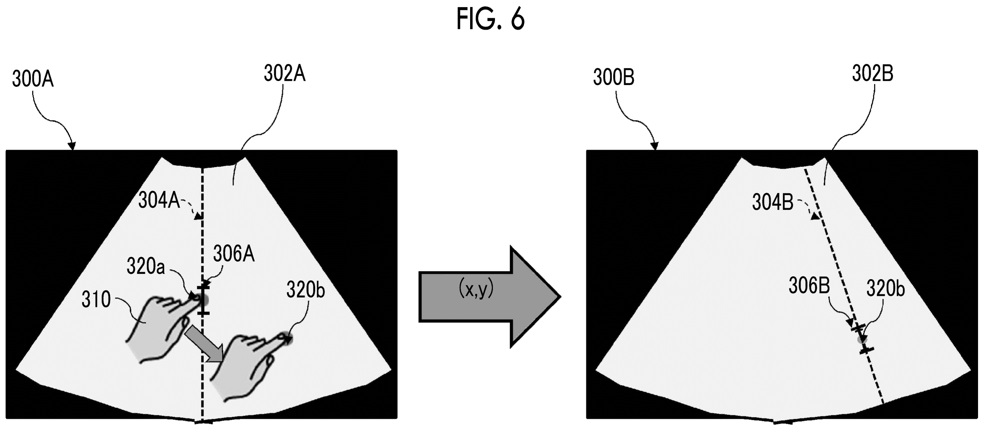

In the related art, the sample volume has been set by using the trackball. In the setting method in the related art, a beamline for setting is displayed on the B-mode tomographic image, and the cursor indicating the sample volume is displayed on the beamline (see a beamline 304A and a cursor 306A in FIG. 6). The displayed beamline is selected from among a plurality of beamlines formed during the scanning with the transmit ultrasound beam by the probe 102. For example, the plurality of beamlines are assigned consecutive numbers in order from the beamline at an end of the scanning range toward the beamline arrangement direction. As the user rotates the trackball in a horizontal direction, the selected beamline number changes by one in the rotation direction. As the user rotates the trackball in the vertical direction, the position of the cursor on the selected beamline moves by one step in the rotation direction. Consecutive numbers are assigned to each step of the depth direction positions, for example, from shallow to deep. For example, an upward rotation moves the cursor shallower on the beamline, and a downward rotation moves the cursor deeper. As described above, in the setting of the sample volume using the trackball in the related art, the number of the beamline on which the cursor is located and the number of the depth direction position of the cursor on the line are sequentially switched by one step in response to the rotation of the trackball.

In addition, in the related art, the width of the sample volume has been changed by rotating a predetermined knob on a console of the ultrasound diagnostic apparatus 100 or by pressing a button on the console to increase or decrease the cursor width.

On the other hand, in the present embodiment, the operation for setting the sample volume is realized by the touch operation on the touch panel on the display unit 118. The specific processing for the setting will be described later.

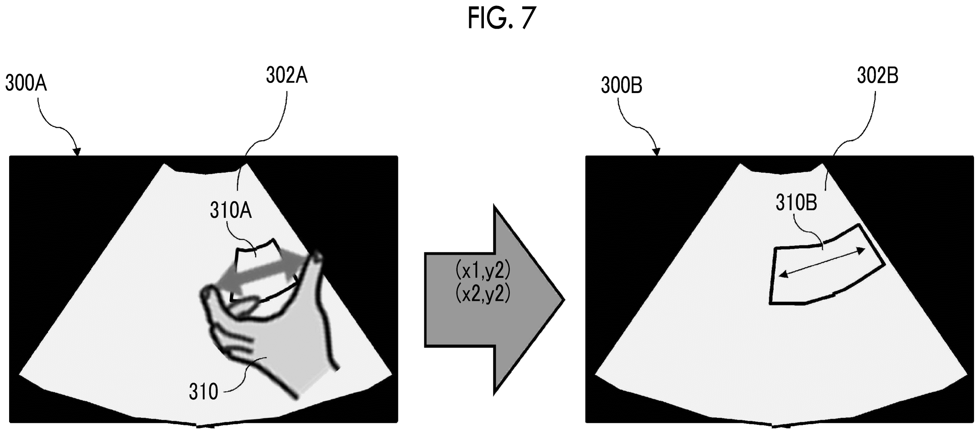

As another example of the control related to the ultrasound transmission and reception, there is control related to the ROI of the color Doppler mode. In the color Doppler mode, due to the speed limit for forming the transmit beam, only a part of the range of the B-mode tomographic image is a target of image formation. The user designates this target range as the ROI. The ROI is determined by positions of both ends in each of the scanning direction and the depth direction in the scanning coordinate system. For example, an ROI 310A shown in FIG. 7 is an example of the ROI in a case in which a convex scanning type probe 102 is used. In a case of the convex scanning, a shape of an ultrasound image 302A is an annular sector, and the ROI 310A of the color Doppler set therein is also an annular sector. Both end edges of the ROI 310A in the scanning direction are along any beamline of the ultrasound beam in the convex scanning. Further, both end edges of the ROI 310A in the depth direction are arcs along the scanning direction.

In the related art, the movement operation of the ROI of the color Doppler has been executed by using the trackball. That is, in a case in which the user selects the ROI on the screen and rotates the trackball, the ROI has been moved by an amount corresponding to the amount of the rotation in the rotation direction. As described above, in the related art, the ROI has been sequentially moved in response to the rotation of the trackball.

In the related art, the size of the ROI has been changed by rotating a knob for ROI size change or pressing an enlargement button or a reduction button for the ROI size.

The ROI has been set in the same manner for the modes other than the color Doppler mode in the related art.

On the other hand, in the present embodiment, the operation for setting the ROI is realized by the touch operation on the touch panel of the display unit 118. The specific processing for the setting will be described later.

Another example of the control executed by the central controller 120 is processing of setting a focus position of the transmit ultrasound beam in response to the designation from the user. In the related art, it has been common to have a function of receiving the designation of the focus depth by operating the knob or the like.

In the present embodiment, the designation of the focus position is received by the touch operation on the display unit 118. In the present embodiment, the focus depth is set to the depth corresponding to the designated focus position. Furthermore, in the present embodiment, the central controller 120 may set a scanning direction position corresponding to the designated focus position as the focus position in the scanning direction. In this case, the central controller 120 sets a beamline group of the transmit ultrasound beam such that the density of the transmit ultrasound beam near the set focus position in the scanning direction is high. An image having a higher resolution than other positions can be obtained at the focus position of the transmit ultrasound beam and the vicinity thereof. The user designates, within the ultrasound image, a position to which the user pays attention, that is, a position of interest, as the focus position.

Hereinafter, the focus in the depth direction will be referred to as a depth direction focus, and the control of increasing the density of the transmit ultrasound beam near the focus position in the scanning direction will be referred to as a scanning direction focus.

The focus control is executed in the scanning coordinate system. The focus position designated on the touch panel is expressed in the display coordinate system. In the present embodiment, the coordinate conversion from the display coordinate system to the scanning coordinate system is executed in order to lead the focus position to the focus control in the scanning coordinate system. The processing of setting the focus position involving the coordinate conversion will be described in detail later.

In order to reflect the touch operation on the touch panel on the display screen in the control of the ultrasound diagnostic apparatus 100, the ultrasound diagnostic apparatus 100 comprises a positional information conversion unit 130 and a control position setting unit 132.

The positional information conversion unit 130 converts a position that is touched by the user on the ultrasound image displayed on the touch panel into coordinates in a coordinate system suitable for the control of the ultrasound diagnostic apparatus 100.

Here, the position on the ultrasound image is, for example, a position in the display coordinate system indicated by the xy coordinates in units of pixels. The coordinates of the touch position on the display screen detected by the touch panel are converted into coordinates (that is, coordinates of the display coordinate system) in the ultrasound image displayed in a window on the display screen by an operating system executed by the central controller 120. The coordinates of the touch position in the display coordinate system are given to the positional information conversion unit 130 as input.

On the other hand, in the control of the ultrasound diagnostic apparatus 100, for example, the control related to the transmit ultrasound beam, the position designation is executed in the scanning coordinate system. The scanning coordinate system is a coordinate system indicated by a combination of coordinates in the scanning direction and coordinates in the depth direction. For example, the coordinates in the scanning direction are indicated by the ultrasound beam numbers, and the coordinates in the depth direction are indicated by the depth direction position numbers.

Further, in another example, the scanning coordinate system is not limited to a set of discretely defined values, such as a beamline number (hereinafter also referred to as "beam number") and a depth direction position number, but may be expressed as a combination of any coordinates in the scanning direction and any coordinates in the depth direction. For example, in a case in which the convex scanning type probe 102 is used, the scanning coordinate system may be a set of coordinates expressed in a polar coordinate form, that is, a form of (r, θ). Here, r indicates any position in the depth direction, that is, a direction away from the origin of the convex scanning, and θ indicates any position in the scanning direction, that is, a circumferential direction centered on the origin of the convex scanning.

The positional information conversion unit 130 converts the information on the touch position on the ultrasound image indicated by the display coordinate system into the positional information in the scanning coordinate system that is easy to use for controlling the ultrasound beam.

The control position setting unit 132 sets the positional information in the scanning coordinate system output by the positional information conversion unit 130 as the positional information used for the control of the ultrasound diagnostic apparatus 100, for example, the control related to the ultrasound beam. Examples of the positional information related to the control of the ultrasound diagnostic apparatus 100 include the position of the cursor of the sample volume, the position of the ROI, and the focus position of the ultrasound beam. The central controller 120 controls the transmission/reception controller 104 and the like in accordance with the set positional information to implement the control in accordance with the position designated by the user by the touch operation.

The positional information conversion unit 130 and the control position setting unit 132 are implemented by executing, for example, a program describing the functions of the components described in the present specification with the processor.

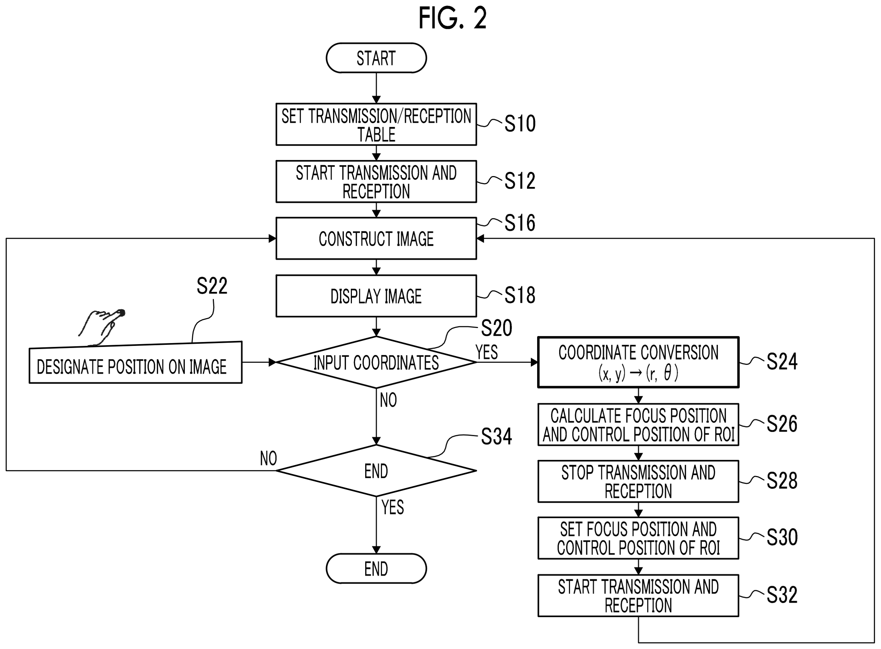

Next, an example of a processing procedure of the present embodiment will be described with reference to FIG. 2.

In the procedure of FIG. 2, in a case in which an instruction to start ultrasound image capturing is issued from the user, the central controller 120 sets a transmission/reception table (S10) and instructs the transmission/reception controller 104 to transmit and receive the ultrasound beam in accordance with the transmission/reception table. As a result, the transmission and reception of the ultrasound beam are started (S12). The transmission/reception table is a table that holds a control parameter of the transmission and reception of the ultrasound beam. Examples of the control parameter held in the transmission/reception table include the focus depth of the transmit beam. In addition, the transmission/reception table may include data of a delay amount to be applied to the transmission signal of each transducer element of the transducer element array in order to implement each of a plurality of selectable transmit focus depths. In a case of the sector type probe or the convex probe, the transmission/reception table may hold data on the delay amount of each transducer element for each direction of the transmit beam (that is, for example, for each beam number).

After the start of the transmission and reception, the ultrasound diagnostic apparatus 100 constructs the ultrasound image such as the B-mode tomographic image by known signal processing based on the reception signal of each transducer element in the probe 102 (S16). The constructed ultrasound image is displayed on the display unit 118 (S18).

While the ultrasound image is being displayed, the central controller 120 determines whether or not there is an input for designating the position coordinates on the ultrasound image, for example, periodically (S20). The user can input the position coordinates on the ultrasound image by, for example, the touch operation on the touch panel of the display unit 118 (S22). The input position coordinates are in the display coordinate system.

In a case in which the position coordinates on the ultrasound image are input (a determination result of S20 is YES), the positional information conversion unit 130 converts the position coordinates into the coordinates in the scanning coordinate system (S24). For example, in a case in which the convex scanning type probe 102 is used, the coordinates (x, y) in the display coordinate system are converted into the coordinates (r, θ) in the scanning coordinate system in the polar coordinate form. The coordinate conversion may be an inverse conversion of the conversion from an operation coordinate system to the display coordinate system executed by the DSC 112.

In this case, r may be a number of the position in the beam direction (that is, the depth direction position), and θ may be a number of the beamline. Here, in a case in which the selectable depth direction position and beamline position in the control of the ultrasound diagnostic apparatus 100 are discrete, there may be a case in which the coordinates (x, y) in the display coordinate system do not completely match the position in (depth direction position, beamline position) that can be taken in the scanning coordinate system. In this case, in the coordinate conversion of step S24, the positional information conversion unit 130 specifies, for the coordinates (x, y) in the display coordinate system, (depth direction position, beamline position) closest to the coordinates (x, y), and determines the number of the depth direction position and the number of the beamline position. The positional information conversion unit 130 outputs the set of the determined number of the depth direction position and the determined number of the beamline as the coordinate conversion results.

It is common that various touch gestures are defined for the touch input with respect to the touch panel. Examples of a one-finger gesture include a tap, a drag, and a flick, and examples of a two-finger gesture include a pinch-in and a pinch-out. In addition, the gesture of simultaneously touching the screen with three or more fingers may be defined. The operating system executed on the central controller 120 determines the type of the input touch gesture and coordinates of one or more touch positions indicating characteristics of the gesture.

For example, in a case of the tap, the tapped coordinates correspond to the coordinates of one or more touch positions indicating the characteristics of the gesture.

In addition, in a case of the drag, for example, coordinates of a start point of the drag, the touch position periodically detected after the start point, and an end point (that is, a position where the finger is finally separated from the screen) correspond to the coordinates of one or more touch positions indicating the characteristics of the gesture. In addition, only the start point and the end point of the drag may be "coordinates of one or more touch positions indicating the characteristics of the gesture".

In addition, in a case of the pinch-in or the pinch-out, two start points that are simultaneously touched, two touch positions that are periodically detected after the start points, and two end points (positions where two fingers are finally separated from the screen) correspond to the coordinates of one or more touch positions indicating the characteristics of the gesture. In addition, only two start points and two end points of the pinch operation may be "coordinates of one or more touch positions indicating the characteristics of the gesture".

In a case of the pinch-in operation, a distance between two fingertips simultaneously touching the touch panel is decreased over time. In a case of the pinch-out operation, the distance between the two fingertips is increased over time.

The positional information conversion unit 130 converts the "coordinates of one or more touch positions indicating the characteristics of the gesture" into the coordinates in the scanning coordinate system. The information on the coordinates in the scanning coordinate system, which is the coordinate conversion result, is transmitted to the control position setting unit 132.

In addition, the information on the type of the touch gesture determined by the central controller 120 is transmitted to the control position setting unit 132.

In addition, the central controller 120 may determine an object (for example, the cursor or the ROI) that is within a predetermined error range from the start point of the user's touch on the screen as the object that is a target of the current operation. In addition, in a case in which there is no object near the single touched point, the central controller 120 may determine that the touch is for designating the focus position.

Next, the control position setting unit 132 calculates a control position in accordance with the coordinates input from the positional information conversion unit 130 and the type of the touch gesture input from the central controller 120 (S26). The control position is a position used to control the transmission or reception of the ultrasound beam. Examples of the control position include the position of the cursor of the sample volume, the position of the ROI, and the focus position of the ultrasound beam.

In a case of the cursor, the position calculated in step S26 may be, for example, a position of a center point of the cursor. In another example, the positions of both ends of the cursor may be calculated. In addition, in a case of the ROI, for example, the position calculated in step S26 may be two points (for example, two points on a diagonal line among the four corner points of the ROI) that uniquely define the position and the size of the ROI. For example, in a case of the drag or the pinch-in and pinch-out operation, coordinates of the end point of a series of operations in the scanning coordinate system are new position coordinates of the object that is the operation target.

In a case in which the calculation in step S26 is completed, the central controller 120 temporarily stops the transmission and reception of the ultrasound beam executed so far by the transmission/reception controller 104 (S28).

Next, the control position setting unit 132 sets the position coordinates calculated in step S26 as the control position corresponding to the touch operation of the user (S30). For example, in a case in which the touch operation of the user is determined to be the designation of the focus position of the transmit ultrasound beam, the control position setting unit 132 sets the position coordinates as the focus position. In addition, in a case in which the object, such as the cursor or the ROI, is selected by the touch operation, the calculated position coordinates are set as new position coordinates of the object. The central controller 120 causes the transmission/reception controller 104 to resume the transmission and reception of the ultrasound in accordance with the set control position (S32). The ultrasound image is constructed and displayed on the display unit 118 in response to the resumed transmission and reception. In a case in which the position coordinates are not input to the touch panel (a determination result in S20 is NO), it is determined whether or not the user has issued an instruction to end the diagnosis (S34). In a case in which the instruction to end the diagnosis is not issued, the processing returns to step S16. In a case in which the instruction to end the diagnosis is issued, the central controller 120 ends the processing shown in FIG. 2.

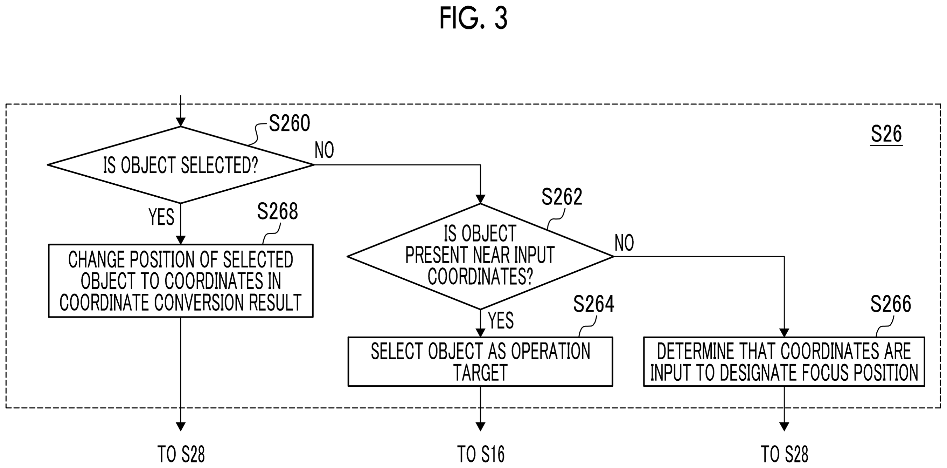

Next, a specific example of the processing of step S26 will be described with reference to FIG. 3. In the example shown in FIG. 3, step S26 includes sub-steps S260 to S266.

In step S260, the control position setting unit 132 determines whether or not the object that is the operation target is selected. In a case in which a determination result is NO, the coordinates touched by the user are the start point of the touch gesture. In this case, it is determined whether or not there is an operable object (for example, the cursor or the ROI) near the coordinates (that is, within the predetermined error range from the coordinates) (S262). The coordinates used in this determination may be a conversion result of the positional information conversion unit 130 or may be coordinates in the display coordinate system before the conversion. In a case in which a determination result in step S262 is YES, the control position setting unit 132 selects the object near the coordinate as the object that is the operation target (S264).

Thereafter, the processing returns to step S16. In this case, the central controller 120 may highlight the selected object that is the operation target on the displayed ultrasound image. The central controller 120 and the control position setting unit 132 continue to detect the touch operation such as the drag on the selected object. In a case in which the touch operation (for example, the drag) continues, the next coordinate conversion result is input from the positional information conversion unit 130. At the time of this input, the object that is the operation target is selected, and a determination result in step S260 is YES. In this case, the control position setting unit 132 changes the position of the selected object that is the operation target to the coordinates indicated by the coordinate conversion result input from the positional information conversion unit 130 (S268). Thereafter, the processing proceeds from step S28 to step S30, and the coordinates of the coordinate conversion result are set as the new coordinates of the object.

In a case in which a determination result in step S262 is NO, there is no operable object near the position touched by the user. In this case, in one example, the control position setting unit 132 determines that the touch operation of the user is the designation of the focus position of the transmit ultrasound beam (S266). Thereafter, the processing proceeds from step S28 to step S30, and the coordinates of the coordinate conversion result are set as the focus position of the transmit ultrasound beam.

In one example, the number of the depth direction position included in the coordinate conversion result is set as the new transmit focus depth. As a result, in the transmission and reception of the ultrasound resumed in step S32, the focus control of the transmit ultrasound beam is executed in accordance with the new setting.

In another example, the transmit focus may be executed in the scanning direction. In this example, for example, control of increasing the density of the transmit ultrasound beam near the scanning direction position indicated by the beam number included in the coordinate conversion result is executed. The number of transmit ultrasound beams that can be formed per frame of the ultrasound image, that is, per scanning, is limited depending on a mode and a desired depth. In the limited number of transmit ultrasound beams, the density of the beam near the position touched by the user is increased. Instead, the number of transmit ultrasound beams per scanning may be maintained by decreasing the density of the beam at a position away from the touch position in the scanning direction. The density referred to here means the number of transmit ultrasound beams per unit length along the scanning direction. In this example, in step S30, the control position setting unit 132 sets the position indicated by the coordinate conversion result received from the positional information conversion unit 130 as the focus position in the scanning direction of the transmit ultrasound beam. In step S32, the central controller 120 sets the beamline of each transmit ultrasound beam such that the density of the transmit ultrasound beam near the set focus position in the scanning direction is increased, and instructs the transmission/reception controller 104 to form the set beamlines. In setting the beamline, for example, the density of the beam at a position away from the focus position may be decreased such that the number of transmit ultrasound beams per scanning is not changed. As a result, the transmission/reception controller 104 controls the transducer element array in the probe 102 to form the designated beamlines.

In addition, the depth direction focus and the scanning direction focus of the transmit ultrasound beam may be executed at the same time.

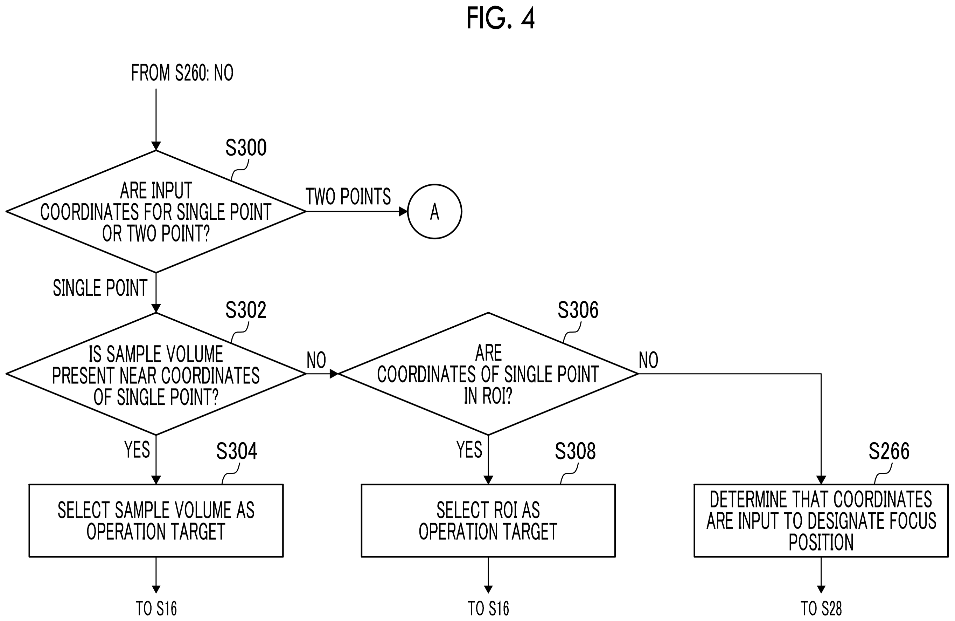

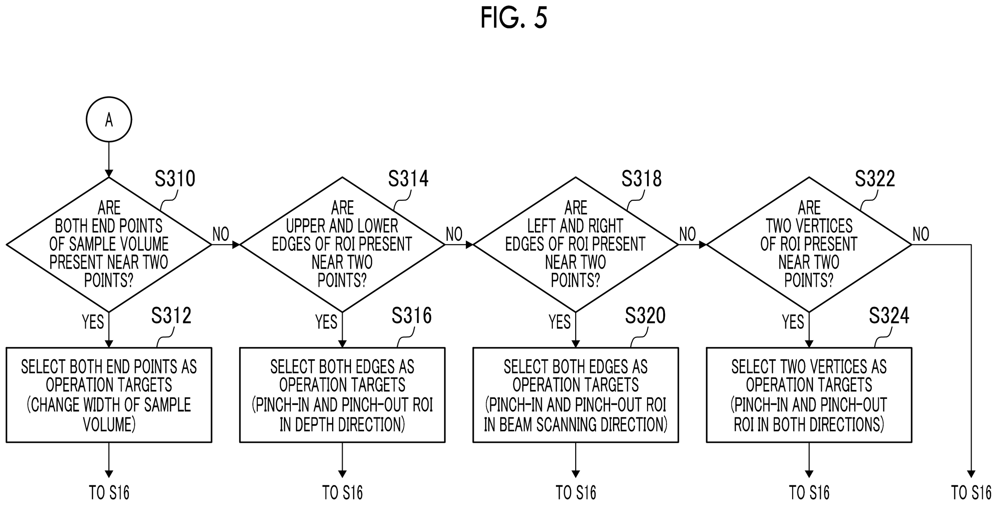

Next, an example of processing of specifying the object as the operation target in response to the touch operation will be described with reference to FIGS. 4 and 5. In this example, the object that is the operation target is specified in consideration of whether the user's operation on the touch panel is single-point touch or two-point touch and the position of the touch. The processing shown in these drawings is processing in a case in which it is determined that the object that is the operation target in FIG. 3 is not yet selected (that is, in a case in which a determination result in step S260 is NO), and is, for example, processing instead of steps S262, S264, and S266 in FIG. 3.

In a case in which a determination result in step S260 is NO, the control position setting unit 132 determines whether the number of points touched at the same time in step S20 in FIG. 3 is a single point or two points (S300). In a case in which a determination result is "single point", it is determined whether or not the cursor of the sample volume is present near the coordinates of the single touched point (S302). In a case in which a determination result is YES, the control position setting unit 132 selects the cursor as the object that is the operation target (S304). In this case, the control position setting unit 132 determines that the user operation is to indicate the position movement of the entire cursor. Thereafter, the central controller 120 and the control position setting unit 132 return to step S16 in FIG. 3 and wait for the instruction of the movement destination of the cursor by the touch operation.

In a case in which a determination result in step S302 is NO, it is determined whether or not the coordinates of the single touched point are located in the ROI or near the ROI (S306). In a case in which a determination result is YES, the control position setting unit 132 selects the ROI as the object that is the operation target (S308). In this case, the control position setting unit 132 determines that the user operation is to indicate the position movement of the entire ROI. Thereafter, the central controller 120 and the control position setting unit 132 return to step S16 in FIG. 3 and wait for the instruction of the movement destination of the ROI by the touch operation.

For example, the movement destination of the cursor or the ROI is designated as the end point of the drag operation. For example, the cursor or the ROI may be selected as the operation target, and the user may touch the screen again to designate the movement destination after temporarily lifting the finger from the screen.

In a case in which a determination result in step S306 is NO, the control position setting unit 132 determines that the coordinates of the single touched point are for designating the focus position of the transmit ultrasound beam (S266 described above).

In a case in which a determination result in step S300 is "two points", the step group shown in FIG. 5 is executed. In the example in FIG. 5, the control position setting unit 132 determines whether or not both end points of the cursor of the sample volume are located near the two points (S310). In a case in which a determination result is YES, the control position setting unit 132 selects both end points of the cursor as the object that is the operation target (S312). After step S312, the processing returns to step S16 of FIG. 3. In this case, the user can change the width of the sample volume by performing the pinch-in or pinch-out with the two fingers that touch the screen. In addition, the cursor movement and the width change can be executed at the same time by performing the drag and the pinch-in or pinch-out with the two fingers.

In a case in which a determination result in step S310 is NO, the control position setting unit 132 determines whether or not an upper edge and a lower edge of the ROI are present near the two points that are touched by the user (S314). The upper edge and the lower edge of the ROI are both end edges of the ROI in the depth direction, and are generally edges extending in the scanning direction. For example, in a case of the convex operation or the sector operation, the upper edge and the lower edge are circular arcs. In a case in which a determination result in step S314 is YES, the control position setting unit 132 selects the upper edge and the lower edge as the operation targets (S316). After step S316, the processing returns to step S16 of FIG. 3. In this case, the user can change the width of the ROI in the depth direction by performing the pinch-in or pinch-out with the two fingers that touch the screen. In addition, the ROI movement and the width change in the depth direction can be executed at the same time by performing the drag and the pinch-in or pinch-out with the two fingers.

In a case in which a determination result in step S314 is NO, the control position setting unit 132 determines whether or not a left edge and a right edge of the ROI are present near the two points that are touched by the user (S318). The left edge and the right edge of the ROI are both end edges of the ROI in the scanning direction, and are generally edges extending in the depth direction. In a case in which a determination result in step S318 is YES, the control position setting unit 132 selects the left edge and the right edge as the operation targets (S320). After step S320, the processing returns to step S16 of FIG. 3. In this case, the user can change the width of the ROI in the scanning direction by performing the pinch-in or pinch-out with the two fingers that touch the screen. In addition, the ROI movement and the width change in the scanning direction can be executed at the same time by performing the drag and the pinch-in or pinch-out with the two fingers.

In a case in which a determination result in step S318 is NO, the control position setting unit 132 determines whether or not there are two vertices on the diagonal line of the ROI near the two points that are touched by the user (S322). In a case in which a determination result in step S322 is YES, the control position setting unit 132 selects the two vertices as the operation targets (S324). After step S324, the processing returns to step S16 of FIG. 3. In this case, the user can enlarge or reduce the ROI in both the scanning direction and the depth direction by performing the pinch-in or pinch-out with the two fingers that touch the screen. In addition, the ROI movement and the enlargement or reduction in both directions can be executed at the same time by performing the drag and the pinch-in or pinch-out with the two fingers.

In a case in which a determination result in step S322 is NO, in the example in FIG. 5, the processing returns to step S16 without selecting the operation target.

The example of the processing of the control position setting unit 132 taking into consideration the single-point touch and the two-point touch has been described above. The behavior of the control position setting unit 132 with respect to the gesture of simultaneously touching three or more points can also be defined.

In the examples shown in FIGS. 4 and 5, the ultrasound diagnostic apparatus 100 determines the intention of the touch, that is, the type of operation that the user wants to perform, in accordance with the number of points touched by the user on the ultrasound image displayed on the touch panel or the positional relationship between the point and the object. However, this configuration is merely an example. Instead, for example, the ultrasound diagnostic apparatus 100 may display a menu indicating the type of operation, such as the movement or the enlargement or the reduction of the cursor, the movement or the enlargement or the reduction of the ROI, or the designation of the focus position, on the screen, and the user may select the type of operation from the menu. In this case, the ultrasound diagnostic apparatus 100 receives the designation of the movement or the enlargement/reduction of the object, the designation of the focus position, or the like by the tap, the drag, the pinch operation, or the like after the selection of the type of the operation.

FIG. 6 shows an example in which the position of the cursor 306A of the sample volume is changed by the touch operation. An image 300A on the left side in FIG. 6 schematically shows a window in which the ultrasound image 302A obtained by the convex scanning at a certain point in time is displayed. An image 300B on the right side schematically shows an image in the same window after the user executes the operation of moving the cursor 306A.

In this example, the PW mode display is also selected, and a blood flow waveform obtained in the PW mode is displayed in a PW mode display window (not shown) outside the image 300A. On the ultrasound image 302A, the cursor 306A representing the sample volume in the PW mode and the beamline 304A on which the sample volume is set are displayed. In a case in which the user wants to examine the blood flow in a portion different from the portion at which the cursor 306A is currently located, the user touches the cursor 306A displayed on the touch panel with a fingertip of a hand 310. A position 320a represents a position touched by the fingertip. The ultrasound diagnostic apparatus 100 determines that the cursor 306A is selected as the operation target because the touched position is located near the cursor 306A. Thereafter, the user drags the touched fingertip to a position 320b of a new sample volume desired by the user. The central controller 120 recognizes, in the display coordinate system, the position 320b of the end point of the drag operation on the ultrasound image 302A, but the positional information conversion unit 130 converts the coordinates into the coordinates in the scanning coordinate system. In the scanning coordinate system, the position 320b is indicated by, for example, a set of the beam number and the number of the depth direction position. This representation is suitable for controlling the ultrasound beam scanning. The coordinates of the position 320b in the scanning coordinate system determined in this manner are set as the position of the sample volume after the movement by the control position setting unit 132. As a result, the cursor 306B located at the position 320b and the beamline 304B on which the cursor 306B is located are displayed on an ultrasound image 302B in the image 300B.

Next, FIG. 7 schematically shows an example in which the size of the ROI is changed by the touch operation. The image 300A on the left side of FIG. 7 schematically shows an image in a window in which the ultrasound image 302A obtained by the convex scanning at a certain point in time is displayed, and the image 300B on the right side schematically shows an image in the same window after the user executes the operation of enlarging the ROI 310A in the scanning direction.

In this example, for example, the ROI 310A in the color Doppler mode is displayed on the ultrasound image 302A. In a case in which the user wants to enlarge the ROI 310A in the scanning direction, the user simultaneously touches the left edge and the right edge of the ROI 310A displayed on the touch panel with fingertips of, for example, a thumb and an index finger of the hand 310. The ultrasound diagnostic apparatus 100 determines that the ROI 310A is selected as the operation target and the content of the scanning is the enlargement or reduction in the scanning direction of the ROI 310A since the positions of the two touched points are located near the left edge and the right edge of the ROI 310A. Thereafter, in a case in which the user wants to enlarge the ROI 310A, the user pinches out two fingertips that are touched in approximately the scanning direction. The central controller 120 recognizes, in the display coordinate system, the position of each end point of the two points of the pinch-out operation on the ultrasound image 302A, but the positional information conversion unit 130 converts these coordinates into the coordinates in the scanning coordinate system. The coordinates in the scanning coordinate system of the positions of two fingertips after the pinch-out operation, particularly the beam number, determined in this manner are set as the positions of both left and right edges of the ROI 310A after the operation. As a result, an ROI 310B enlarged in the scanning direction as compared to that in the image 300A is displayed on the ultrasound image 302B in the image 300B.

In the above description, the ROI in the color Doppler mode has been described as one of representative examples. The ROI is set in the B-mode tomographic image, and defines a range in which the ultrasound beams are transmitted and received for generating the color Doppler image and a range in which the color Doppler image is generated from the reception signals obtained by the transmission and reception. However, the ROI in the color Doppler mode is merely an example. There are also display modes, in which the ROI indicating the target range is set, other than the color Doppler mode and the above-described processing can be applied to the ROI in these other display modes.

In addition, the ultrasound diagnostic apparatus 100 according to the present embodiment may receive the enlargement or reduction of the scanning range (that is, the width in the scanning direction) of the transmit ultrasound beam for the B-mode tomographic image by the touch operation. The operation for the enlargement or reduction in this case or the processing corresponding to the operation may be the same as in a case of the enlargement or reduction in the scanning direction of the ROI. For example, in a case in which the scanning range is reduced, the number of transmit ultrasound beams per scanning is reduced, so that the time required for one scanning is shortened, and thus the frame rate of the B-mode tomographic image to be displayed can be increased.

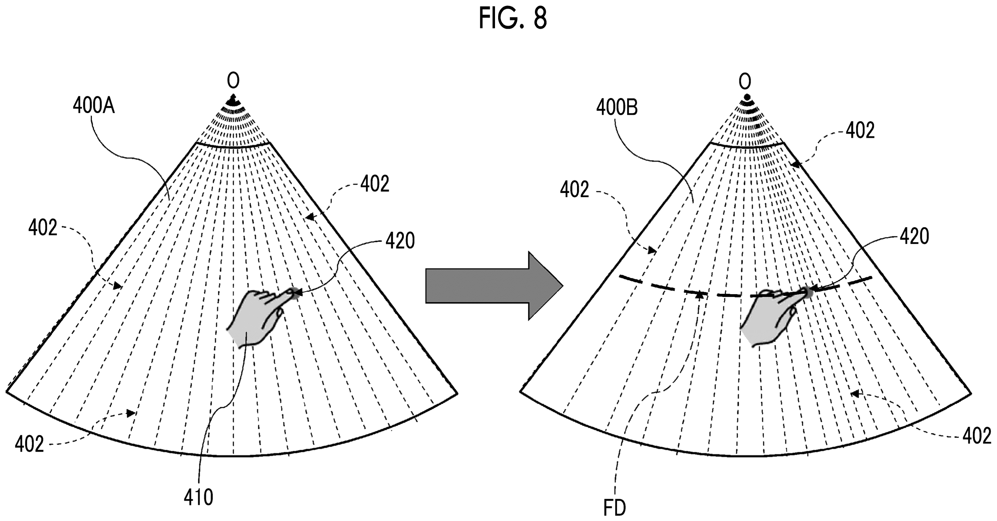

Next, an example in which the size of the ROI is changed by the touch operation will be described with reference to FIG. 8. An ultrasound image 400A on the left side in FIG. 8 schematically shows an ultrasound image obtained by the convex scanning at a certain point in time, and an ultrasound image 400B on the right side schematically shows an ultrasound image in a case in which control is executed in response to the designation of the transmit focus position from the user. The ultrasound image 400A is an image obtained by executing the transmit beam control in the setting in which the focus is set to a default focus depth in the depth direction and the focus is not set in the scanning direction. A dashed line 402 extending radially from an origin O of the convex scanning indicates the beamline of the transmit ultrasound beam. Since the scanning direction focus is not executed, the dashed lines 402 indicating the beamlines are arranged at equal intervals along the scanning direction.

In a case in which the user wants to examine a location in the ultrasound image 400A in detail, the user touches a point 420 in the location with a fingertip of a hand 410. Since the touched point 420 is within the ultrasound image 400A and there is no object such as the cursor or the ROI near the point 420, the ultrasound diagnostic apparatus 100 determines that the current operation of the user is to designate the focus position. The positional information conversion unit 130 converts the coordinates of the point 420 into the coordinates in the scanning coordinate system, for example, a set of the beam number and the number of the depth direction position. The control position setting unit 132 sets the determined coordinates in the scanning coordinate system as the focus position of the transmit beam. For example, the number of the depth direction position of the coordinates is set to the focus depth, and the beam number is set to the focus position in the scanning direction. The central controller 120 sets the beamline of the transmit beam such that, for example, the density of the transmit beam is maximized at the focus position in the scanning direction and the density of the transmit beam is decreased as the position is away from the focus position in the scanning direction, in accordance with the setting of the focus position in the scanning direction. Then, the transmission/reception controller 104 is instructed to transmit the ultrasound beam in accordance with these settings.

In the transmission of the ultrasound beam by the transmission/reception controller 104 in response to the instruction, as schematically shown on the ultrasound image 400B, the depth direction position of the point 420 touched by the user is a focus depth FD. Since the convex scanning is used in this example, an arc passing through the point 420 and having the origin O as the center is a line of the focus depth FD. In addition, the beamline (indicated by the dashed line 402) of the transmit beam is set to be higher in density as the position is closer to the point 420 in the scanning direction and to be lower in density as the position is farther from the point 420.

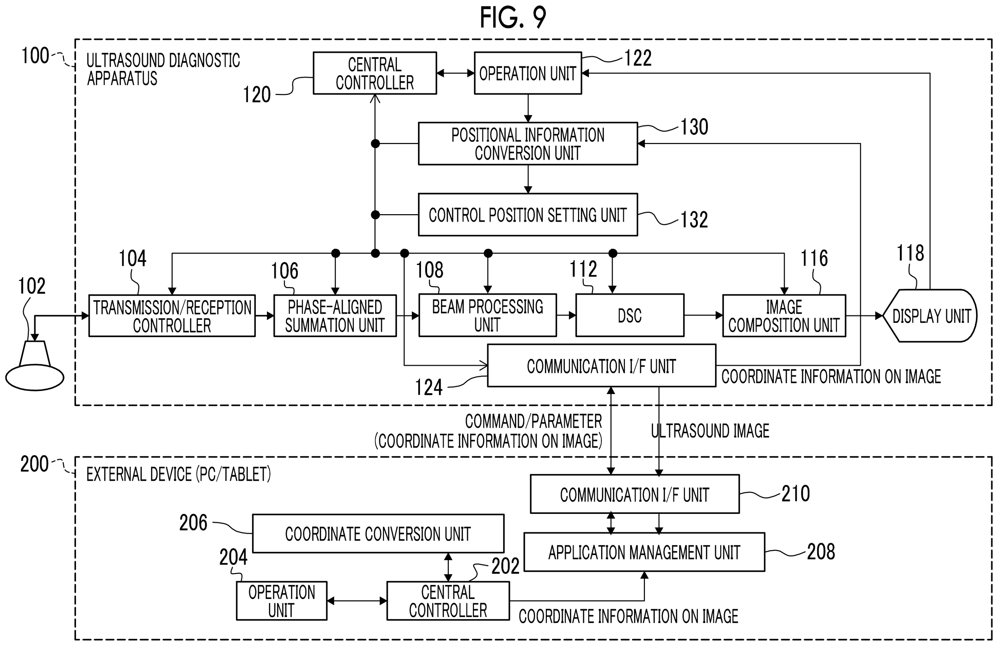

Next, a modification example of the present embodiment will be described with reference to FIG. 9. In the modification example, the ultrasound diagnostic apparatus 100 is connected to an external device 200 such as a tablet terminal or a personal computer. The external device 200 incorporates a display device comprising a touch panel. The display unit 118 functions as the UI screen in the embodiment shown in FIG. 1, whereas the screen of the display device of the external device 200 functions as the UI screen in this modification example.

The ultrasound diagnostic apparatus 100 comprises a communication interface (I/F) unit 124 in addition to the components shown in FIG. 1. The communication I/F unit 124 is an interface for data communication with the external device 200. The data communication between the ultrasound diagnostic apparatus 100 and the external device 200 may be wireless or wired.

The external device 200 comprises a central controller 202, an operation unit 204, a coordinate conversion unit 206, an application management unit 208, and a communication I/F unit 210.

The central controller 202 is a computer that controls the operation of the external device 200. The central controller 202 includes hardware such as a CPU, a memory, and a large-capacity storage device, and software such as an OS. The operation unit 204 is a user interface device of the external device 200.

In one example, the operation unit 204 comprises a touch panel display, displays a screen including the ultrasound image transmitted from the ultrasound diagnostic apparatus 100, and detects the touch of the user on the screen. The touch panel detects coordinates of the touch position in a coordinate system of the panel. The coordinate conversion unit 206 converts the coordinates into the coordinates in the coordinate system of the ultrasound image displayed on the touch panel, that is, the coordinates in the display coordinate system. The coordinate conversion is realized as a function of the OS, for example. The application management unit 208 manages the execution of the application on the external device 200. Among the applications to be executed, there is an application (hereinafter, referred to as an "ultrasound application") that executes processing to function as a UI of the ultrasound diagnostic apparatus 100. The ultrasound application displays, for example, the ultrasound image input from the ultrasound diagnostic apparatus 100 in a predetermined window on the UI screen displayed on the touch panel. In addition, the ultrasound application transmits, to the ultrasound diagnostic apparatus 100 via the communication I/F unit 210, a command or a parameter input by the user to the UI screen by the touch operation, such as the coordinates of the touch position in the display coordinate system output by the coordinate conversion unit 206, in response to the touch of the user. The communication I/F unit 210 is an interface for data communication with the ultrasound diagnostic apparatus 100.

In this modification example, the ultrasound diagnostic apparatus 100 transmits the generated ultrasound image to the external device 200 via the communication I/F unit 124. The ultrasound image is transmitted, for example, by streaming. The ultrasound application of the external device 200 displays the ultrasound image on the UI screen and receives the touch input of the user to the UI screen. In a case in which the user touches any position on the ultrasound image on the UI screen, the coordinate conversion unit 206 converts the touch position into the coordinates in the display coordinate system of the ultrasound image. The coordinates of the conversion result are transmitted to the ultrasound diagnostic apparatus 100 via the application management unit 208 and the communication I/F unit 210.

In the ultrasound diagnostic apparatus 100, the positional information conversion unit 130 converts the coordinates into the coordinates in the scanning coordinate system. Then, the control position setting unit 132 sets the positional information for controlling the ultrasound diagnostic apparatus 100 by using the coordinate conversion result. Then, the central controller 120 controls the operation of the ultrasound diagnostic apparatus 100, for example, the transmission of the ultrasound, in accordance with the setting. The processing of the setting and the control is the same as the processing in the above-described embodiment described with reference to FIGS. 2 to 5.

In the above description, the example has been described in which the touch panel is used as the device that inputs the position on the screen, but the processing in the present embodiment can be applied to a case in which a pointing device (for example, a mouse) other than the touch panel is used.

In the embodiment described above, the width of the ROI or the cursor is an example of the range used to generate the ultrasound image, and is also an example of the range that defines the transmission range of the ultrasound beam for a specific display mode. In addition, the position of the cursor or the position of the transmit beam focus is an example of the position used to generate the ultrasound image, and is also a range that defines the transmission position of the ultrasound beam for a specific display mode. In addition, the ROI or the cursor can also be said to be a range that defines the range of the reception signal that is the operation target for generating the image in a specific display mode of the ultrasound diagnostic apparatus.

In addition, the control of the depth direction focus and the scanning direction focus of the transmit ultrasound beam can also be applied to a method in which the focus position is designated using a trackball, an arrow key, or the like. That is, in this method, for example, as in a case of designating the cursor position of the sample volume in the related art, the focus position is designated while being switched stepwise in the depth direction and/or the scanning direction by using the trackball or the arrow key. For example, when the right (or left) arrow key is pressed once, the focus position is moved by one step to the right (or left) side along the scanning direction from the current position. The movement amount of the focus position is an amount corresponding to the number of times the right arrow key is pressed. The up and down arrow keys are used to indicate the movement of the focus position in the depth direction. The same applies to the operation using the trackball. For example, in a case in which the trackball is rotated to the right, the focus position is moved by an amount corresponding to the amount of rotation to the right side along the scanning direction from the current position. In addition, in a case in which the trackball is rotated downward, the focus position is moved by an amount corresponding to the amount of rotation in the deeper direction. As a result, the beam number and the number of the depth direction position for specifying the focus position are designated. The control position setting unit 132 sets the designated depth direction position as the focus depth, and sets the designated beam number as the focus position in the scanning direction.

In the present embodiment, each type of processing is executed by any computer. In addition, any computer may execute these types of processing by a processor as hardware, a program as software, or a combination thereof. In such a case, the processor is configured to execute various types of processing in the present embodiment in cooperation with the program, and may function as each unit or each means in the present embodiment. In addition, the execution order of the processing by the processor is not limited to the above-described order and may be changed as appropriate. Any computer may be a general-purpose computer, a computer for specific use, a workstation, or another system that can execute each type of processing.

The processor may be configured by one or more types of hardware, and the type of hardware is not limited. For example, the processor may be implemented by hardware such as a programmable logic device, for example, a central processing unit (CPU), a micro processing unit (MPU), or a field programmable gate array (FPGA), a dedicated circuit for executing specific processing such as an application specific integrated circuit (ASIC), a graphics processing unit (GPU), or a neural processing unit (NPU). Moreover, the kind of hardware may be a combination of different types of hardware. In a case in which the plurality of types of hardware are configured to execute one or a plurality of types of processing of a certain processor, the plurality of types of hardware may be present in devices physically separated from each other or may be present in the same device. Furthermore, in any of the embodiments, the order of each type of processing executed by the processor is not limited to the above-described order, and may be changed as appropriate. In addition, hardware is implemented in a form of an electric circuit (circuitry) in which circuit elements, such as semiconductor elements, are combined.

Furthermore, the program may be software such as firmware or microcode. The program may be, for example, a group of program modules, and each function thereof may be implemented by a processor configured to execute each function. The program may be a program code or a plurality of code segments stored in one or more non-transitory computer-readable media (for example, a storage medium and other storages). The program may be stored in the plurality of non-transitory computer-readable media present in devices physically separated from each other. The program code or the code segment may represent any combination of procedures, functions, subprograms, routines, subroutines, modules, software packages, classes, instructions, data structures, or program statements. The program code or the code segment may be connected to another code segment or a hardware circuit by transmitting and receiving information, data, arguments, parameters, or contents in the memory.

### Explanation of References

100: ultrasound diagnostic apparatus
102: probe
104: transmission/reception controller
106: phase-aligned summation unit
108: beam processing unit
110: data memory
112: digital scan converter (DSC)
114: image memory
116: image composition unit
118: display unit
120: central controller
122: operation unit
130: positional information conversion unit
132: control position setting unit

## Claims

1. An ultrasound diagnostic apparatus comprising:
a processor configured to:
receive information on a point designated on an ultrasound image displayed on a screen;
perform coordinate conversion of coordinates of the designated point in a display coordinate system of the ultrasound image that are indicated by the information into coordinates in a scanning coordinate system of an ultrasound beam; and
execute control of the ultrasound diagnostic apparatus using the coordinates of the designated point that have undergone the coordinate conversion.

2. The ultrasound diagnostic apparatus according to claim 1,
wherein the processor is configured to:
in the control, set a range or a position that is used to generate the ultrasound image based on the coordinates of the designated point that have undergone the coordinate conversion.

3. The ultrasound diagnostic apparatus according to claim 2,
wherein the range or the position that is used to generate the ultrasound image and that is a target of the setting defines a transmission range or a transmission position of the ultrasound beam for a specific display mode of the ultrasound diagnostic apparatus.

4. The ultrasound diagnostic apparatus according to claim 2,
wherein the range that is used to generate the ultrasound image and that is a target of the setting defines a range of reception signals subjected to calculation for generating an image of a specific display mode of the ultrasound diagnostic apparatus.

5. The ultrasound diagnostic apparatus according to claim 3 or 4,
wherein the range that is used to generate the ultrasound image and that is the target of the setting is a sample volume for pulsed Doppler display.

6. The ultrasound diagnostic apparatus according to claim 3 or 4,
wherein the range that is used to generate the ultrasound image and that is the target of the setting is an ROI that is a range for color Doppler display.

7. The ultrasound diagnostic apparatus according to claim 3 or 4,
wherein the range that is used to generate the ultrasound image and that is the target of the setting is a scanning range with the ultrasound beam for B-mode tomographic image display.

8. The ultrasound diagnostic apparatus according to claim 2,
wherein the processor is configured to:
set the range that is used to generate the ultrasound image based on the designated point on the ultrasound image displayed on the screen.

9. The ultrasound diagnostic apparatus according to any one of claims 2 to 8,
wherein the processor is configured to:
receive information on two points designated on the ultrasound image displayed on the screen; and
enlarge or reduce the range in a case in which a distance between the two designated points is increased or decreased over time.

10. A program causing a computer to execute a process comprising:
receiving information on a point designated on an ultrasound image displayed on a screen;
performing coordinate conversion of coordinates of the designated point in a display coordinate system of the ultrasound image that are indicated by the information into coordinates in a scanning coordinate system of an ultrasound beam; and
executing control of the ultrasound diagnostic apparatus using the coordinates of the designated point that have undergone the coordinate conversion.

11. A method for controlling an ultrasound diagnostic apparatus comprising:
receiving information on a point designated on an ultrasound image displayed on a screen;
performing coordinate conversion of coordinates of the designated point in a display coordinate system of the ultrasound image that are indicated by the information into coordinates in a scanning coordinate system of an ultrasound beam; and
executing control of the ultrasound diagnostic apparatus using the coordinates of the designated point that have undergone the coordinate conversion.
